# EUROPEAN PATENT SPECIFICATION

(11) **EP 1 415 644 B1**
(45) Date of publication and mention of the grant of the patent: **20.02.2008**
(21) Application number: 03292457.3
(22) Date of filing: 03.10.2003
(51) Int. Cl.: A61K 8/97, A61Q 99/00

(54) **Cosmetic process for smoothing out expression wrinkles by topical application of a perfume composition**
Kosmetisches Verfahren zur Glättung von Expressionsfalten durch topisches Aufbringen einer Parfümzusammensetzung
Procédé cosmétique de lissage des rides d'expression par application topique d'une composition de parfum

(30) Priority: 29.10.2002 FR 0213523
(43) Date of publication of application: 06.05.2004
(73) Proprietor: L'ORÉAL, 75008 Paris (FR)
(72) Inventor: Abriat, Anne, 75016 Paris (FR); Petit, Brigitte, 75007 Paris (FR)
(74) Representative: Leszczynski, André

(56) References cited:
- WO-A-00/66078
- WO-A-02/34228
- WO-A-99/36052
- FR-A- 2 631 824
- PATENT ABSTRACTS OF JAPAN vol. 2000, no. 20, 10 July 2001 (2001-07-10) & JP 2001 064193 A (OKADA SAIJI), 13 March 2001 (2001-03-13)

## Description

The present invention relates to a cosmetic process for preventing and/or smoothing out expression wrinkles and/or for relaxing the lines of the face, comprising the topical application to facial skin of a cosmetic composition containing, in a physiologically acceptable medium, a fragrancing composition of given constitution, this fragrancing composition resulting in, when it is introduced in a proportion of 0.5% by weight into 2 ml of a topical preparation applied to the part of the skin located between the nose and the top lip of a human individual subjected to stress, a reduction in the muscular activity of the trapezius muscle, measured by electromyography, compared with the unfragranced topical preparation tested under the same conditions.

Women, and even men, currently have a tendency to wish to look youthful for as long as possible and consequently seek to fade out the signs of ageing on the skin, which are reflected in particular by wrinkles and fine lines. In this respect, the media and the fashion world report about products intended to keep the skin radiant and wrinkle-free for as long as possible, which are signs of youthful skin, and all the more so since the physical appearance acts on the psyche and/or on the morale.

Hitherto, wrinkles and fine lines were treated using cosmetic products containing active agents acting on the skin, for example by moisturizing it or by improving its cell renewal or alternatively by promoting the synthesis of collagen, of which skin tissue is composed. WO 02/34228 proposes to treat variables by topical action of myorelaxing agents.

Although these treatments make it possible to act on the wrinkles and fine lines caused by chronological or intrinsic ageing, and also on those caused by photoageing, they have no effect on expression wrinkles and fine lines, which require an intervention on the contractile muscle component of the wrinkles present in the skin.

Hitherto, the only means commonly used for acting on expression wrinkles is botulinum toxin, which is especially injected into the wrinkles of the glabella, which are the wrinkles between the eyebrows (see J.D. Carruters et al., J. Dermatol. Surg. Oncol., 1992, 18, pp. 17-21).

The Applicant has also proposed various compounds capable of affording a muscle-relaxing effect when they are applied topically to the skin, thus making it possible to act on expression wrinkles via another route. Among these compounds, mention may be made especially of antagonists of the receptors associated with the calcium channels (FR-2 793 681), and in particular manganese and its salts (FR-2 809 005) and alverine (FR-2 798 590); and agonists of the receptors associated with the chlorine channels, including glycine (EP-0 704 210) and certain extracts of *Iris pallida* (FR-2 746 641).

However, these natural compounds and extracts are not always easy to formulate in anti-ageing compositions.

The Applicant has now discovered, surprisingly, that certain fragrancing compositions have relaxing properties on certain striated muscles, making it possible to envisage their use for relaxing the muscles of the face and thus their introduction into cosmetic compositions for combating expression wrinkles.

To the Applicant's knowledge, it has never as yet been proposed to use fragrances for this purpose, despite the development of aromatherapy.

One subject of the invention is thus a cosmetic process via inhalation of a fragrancing composition for preventing and/or smoothing out expression wrinkles and/or for relaxing the lines of the face, comprising the topical application to facial skin of a cosmetic composition containing, in a physiologically acceptable medium said fragrancing composition, said fragrancing composition containing from 5% to 10% by weight of essential oils and from 90% to 95% by weight of a mixture comprising:
(a) from 10% to 15% by weight of alcohols,
(b) from 10% to 15% by weight of aldehydes,
(c) from 25% to 30% by weight of esters,
(d) from 20% to 30% by weight of at least one compound chosen from: musks and ketones, and
(e) from 5% to 10% by weight of solvents,
said essential oils being chosen among the essential oils of lemon, of orange, of aniseed, of bergamot, of rose, of geranium, of ginger, of neroli, of basil, of rosemary, of cardamom, of camphor, of cedar, of camomile, of sandalwood, and of sage, and mixtures thereof and a mixture of bergamot, mandarin and ylang-ylang,
said compounds (a) to (d) being chosen among geraniol, geranyl acetate, farnesol, borneol, bornyl acetate, linalool, linalyl acetate, linalyl propionate, linalyl butyrate, tetrahydrolinalool, citronellol, citronellyl acetate, citronellyl formate, citronellyl propionate, dihydromyrcenol, dihydromyrcenyl acetate, tetrahydromyrcenol, terpineol, terpinyl acetate, nopol, nopyl acetate, nerol, neryl acetate, 2-phenylethanol, 2-phenylethyl acetate, benzyl alcohol, benzyl acetate, benzyl salicylate, styrallyl acetate, benzyl benzoate, amyl salicylate, dimethylbenzylcarbinol, trichloromethylphenylcarbinyl acetate, p-tert-butylcyclohexyl acetate, isononyl acetate, vetiveryl acetate, vetiverol, α-hexylcinnamaldehyde, 2-methyl-3-(p-tert-butylphenyl)-propanal,
- methyl-3-(p-isopropylphenyl)propanal, 3-(p-tert-butylphenyl)propanal, 2,4-dimethylcyclohex-3-enylcarboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyltetrahydropyran, 3-carboxymethyl-2-pentylcyclopentane, 2-n-heptylcyclopentanone, 3-methyl-2-pentyl-2-cyclopentenone, menthone, carvone, tagetone, geranyl acetone, n-decanal, n-dodecanal, 9-decen-1-ol, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepinonitrile, aubepine, heliotropin, coumarin, eugenol, vanillin, diphenyl ether, citral, citronellal, hydroxycitronellal, damascone, ionones, methylionones, isomethylionones, solanone, irones, cis-3-hexenol and its esters, indane musks, tetralin musks, isochroman musks, macrocyclic ketones, macrolactone musks and ethylene brassylate, and mixtures thereof
   and said solvents being chose among ethanol, isopropanol, diethylene glycol monoethyl ether, dipropylene glycol, diethyl phthalate, triethyl citrate and isopropyl myristate,
   this fragrancing composition resulting in, when it is introduced in a proportion of 0.5% by weight into 2 ml of a topical preparation applied to the part of the skin located between the nose and the top lip of a human individual subjected to stress, a reduction in the muscular activity of the trapezius muscle, measured by electromyography, compared with the unfragranced topical preparation tested under the same conditions.

The electromyography test, which makes it possible to select the fragrancing compositions that may be used according to the invention, is described in greater detail in Example 1. To perform this test, the fragrancing composition is formulated in a topical preparation, which may be of any chemical composition provided that this preparation is also used as a control in this test. The same reference topical preparation may be used to test different fragrancing compositions. In contrast with the cosmetic composition according to the invention, the topical preparation does not necessarily satisfy all the requirements of a commercial cosmetic composition, in particular as regards its texture and its stability over time.

The cosmetic composition used according to the invention is suitable for topical application to the skin and thus contains a physiologically acceptable medium, i.e. a medium that is compatible with the skin and optionally with its integuments (eyelashes, nails and hair) and/or mucous membranes. It may be a skincare, cleansing or makeup composition.

It contains an amount of fragrancing composition that is sufficient to obtain the desired effect and preferably from 0.1% to 25% by weight relative to the total weight of the cosmetic composition.

This composition is advantageously applied to individuals with expression wrinkles and fine lines. These wrinkles and fine lines are mainly located radially around the mouth and/or the eyes and/or horizontally on the forehead and/or in the space between the eyebrows. In addition, since its beneficial effect on the skin acts via inhalation of the fragrancing composition it contains, the cosmetic composition according to the invention is also preferably applied to the part of the individual's skin located between the nose and the mouth.

The term "fragrancing composition" used herein denotes a mixture of odoriferous materials whose vapour pressure is less than the atmospheric pressure at 25°C and which are generally liquid at 25°C.

The constituents of the fragrancing composition used according to the invention are compounds usually used by perfumers and are described especially in S. Arctander, Perfume and Flavor Chemicals (Montclair, N.J., 1969), in S. Arctander, Perfume and Flavor Materials of Natural Origin (Elizabeth, N.J., 1960) and in "Flavor and Fragrance Materials - 1991", Allured Publishing Co. Wheaton, III. USA.

They may be natural products (essential oils, absolutes, resinoids, resins or concretes) and/or synthetic products (hydrocarbons, alcohols, aldehydes, ketones, ethers, acids, esters, acetals, ketals, nitriles, which may be saturated or unsaturated, and aliphatic or cyclic).

The fragrancing composition according to the invention also comprises solvents that are standard in fragrances, which may be chosen especially from: ethanol, isopropanol, diethylene glycol monoethyl ether, dipropylene glycol, diethyl phthalate, triethyl citrate, isopropyl myristate.

The cosmetic composition containing the fragrancing composition according to the invention may be in any presentation form conventionally used for topical application and especially in the form of aqueous gels and aqueous or aqueous-alcoholic solutions. By adding a fatty or oily phase, it may also be in the form of dispersions of the lotion or serum type, emulsions of liquid or semi-liquid consistency of the milk type, obtained by dispersing a fatty phase in an aqueous phase (O/W) or conversely (W/O), or suspensions or emulsions of soft, semi-solid or solid consistency of the cream or gel type, or multiple emulsions (W/O/W or O/W/O emulsions), microemulsions, vesicular dispersions of ionic and/or nonionic type, or wax/aqueous phase dispersions. These compositions are prepared according to the usual methods.

According to one preferred embodiment of the invention, the cosmetic composition is in the form of an emulsion.

In this case, the proportion of the oily phase of the emulsion may range, for example, from 5% to 80% by weight and preferably from 5% to 50% by weight relative to the total weight of the composition. The fatty substances, emulsifiers and co-emulsifiers used in the composition in emulsion form are chosen from those conventionally used in cosmetics or dermatology. The emulsifier and the co-emulsifier are generally present in the composition in a proportion ranging from 0.3% to 30% by weight and preferably from 0.5% to 20% by weight relative to the total weight of the composition. The emulsion may also contain lipid vesicles.

Fatty substances that may be used include oils and especially mineral oils (liquid petroleum jelly), oils of plant origin (avocado oil, rice bran oil, wheatgerm oil, apricot kernel oil, soybean oil, coconut oil, palm oil and rapeseed oil), oils of animal origin (lanolin), synthetic oils (perhydrosqualene and hydrogenated polyisobutene), silicone oils (cyclomethicone) and fluoro oils (perfluoropolyethers). Fatty substances that may also be used include fatty alcohols such as cetyl alcohol, stearyl alcohol and octyldodecanol, fatty acids, fatty acid esters such as pentaerythrityl tetraethylhexanoate, waxes and gums, and in particular silicone gums.

Examples of emulsifiers and co-emulsifiers that may be mentioned include fatty acid esters of polyethylene glycol, such as PEG-100 stearate, PEG-40 stearate and PEG-20 stearate; fatty acid esters of polyols, such as glyceryl stearate, sorbitan tristearate and the oxyethylenated sorbitan stearates sold under the trade names Tween^{®} 20 or Tween^{®} 60, for example; and mixtures thereof.

In a known manner, the cosmetic composition used according to the invention may also contain adjuvants that are common in cosmetics, such as hydrophilic or lipophilic gelling agents, hydrophilic or lipophilic active agents, preserving agents, fragrances, fillers, screening agents, pigments, odour absorbers and dyestuffs. The amounts of these various adjuvants are those conventionally used in the field under consideration, for example from 0.01% to 20% of the total weight of the composition. Depending on their nature, these adjuvants may be introduced into the fatty phase, into the aqueous phase or into the lipid vesicles. In any case, these adjuvants and the proportions thereof will be chosen so as not to harm the desired properties of the fragrancing composition according to the invention.

Hydrophilic gelling agents that may be mentioned in particular include carboxyvinyl polymers (carbomer), acrylic copolymers such as acrylate/alkylacrylate copolymers, polyacrylamides and in particular acrylamide/sodium acryloyldimethyltaurate copolymer and the ammonium salt of polyacryloyldimethyl taurate, polysaccharides, natural gums and clays, and lipophilic gelling agents that may be mentioned include modified clays, for instance bentones, metal salts of fatty acids and hydrophobic silica.

Fillers that may be mentioned especially include talc and silica, and also polyamide fibres.

As active agents, the cosmetic composition according to the invention also advantageously contains at least one compound that may be chosen especially from: desquamating agents; moisturizers; depigmenting or propigmenting agents; anti-glycation agents; NO-synthase inhibitors; agents for stimulating the synthesis of dermal or epidermal macromolecules and/or for preventing their degradation; agents for stimulating fibroblast and/or keratinocyte proliferation or for stimulating keratinocyte differentiation; muscle relaxants; tensioning agents; antipollution agents and/or free-radical scavengers; agents acting on the capillary circulation; agents acting on the energy metabolism of cells; and mixtures thereof.

Active agents that are preferred for use according to the present invention comprise one or more compounds chosen from: manganese gluconate; an extract of wild yam containing diosgenin; a ceramide; a retinoid chosen from: retinyl palmitate, retinyl caprylate and retinol; and acetyl trifluoromethylphenyl valylglycine.

### EXAMPLES

### Example 1: Demonstration of the relaxing effect on striated muscle

### Experimental protocol

The effect on the trapezius muscle of a fragrancing composition formulated in a topical preparation in cream form was evaluated.

The fragrancing composition tested consisted of a mixture of:
- natural products 7%, including essential oil of bergamot, mandarin and ylang-ylang
- synthetic products 93%, including
   - aliphatic and aromatic alcohols 13%
   - aliphatic and aromatic aldehydes 13.50%
   - aliphatic and aromatic esters 28.50%
   - synthetic ketones and musks 25%
   - solvents 9%
   - various (monoterpenes, saturated products) 4%

The study was performed on 40 individuals between 21 and 50 years old. The individuals were divided into 2 groups of 20 individuals. One group tested the fragranced cream and the second group the unfragranced base. The fragrance presented was of good intensity, neither too strong nor too weak.

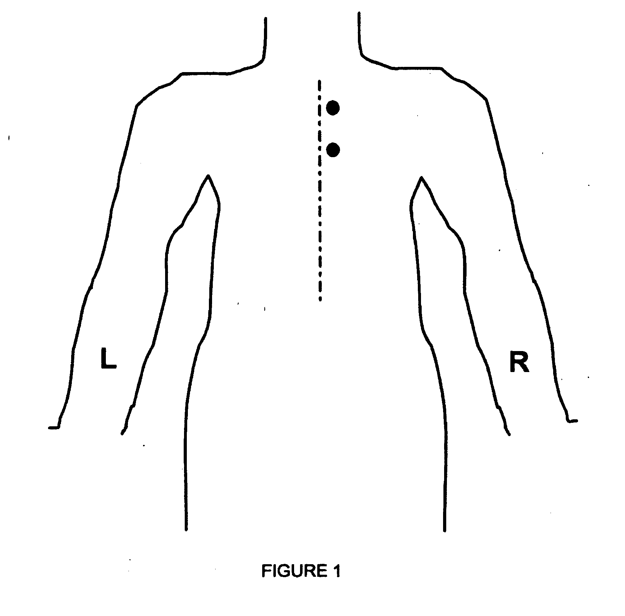

Sensors coated with conductive gel were placed on the individuals according to the scheme indicated in Figure 1 attached hereto, in which the dotted line illustrates the vertebral column of the individual and the discs denote the positions of the sensors, the individual being viewed from the back. The target areas of skin were wiped with alcohol before application, to improve the signal-to-noise ratio.

After the sensors were placed on the individuals, the said individuals were subjected to a first series of tests lasting 30 minutes to familiarize them with the procedure. Each individual then applied 2 ml of cream by finger to the area of skin located between the top lip and the nose.

The activity of the trapezius muscle of each individual was measured by electromyography using sensors installed beforehand, with the aid of a Physiolab I-330 C-2 machine from J&J Engineering Inc. during each of the three-minute periods below:
■ first period (rest): the individual was placed, with the eyes open, before a pleasant scene
■ second period (training): the individual had to count coloured squares following each other every 10 seconds
■ third period (stress): the individual had to name the colours of series of words passing before him at a rate of one per second, the words corresponding to colours different from those in which they were written. In the middle of the test, this was complicated by moving the word on the screen and adding stressful sounds such as breaking glass, the screeching of tyres, etc.
■ fourth period (recovery): as for the second period.

The data were filtered using a 100-400 Hz filter and then integrated and their mean was calculated to obtain a mean value in microvolts corresponding to each of the above four periods.

The value corresponding to the first period, which is not significantly different from one group to another, was used as covariance to eliminate the differences between the groups and between the individuals. This made it possible to obtain a finer statistical analysis and to give a better analysis of the effects. The EMG data were analysed by means of repeated covariance measurements (ANCOVA) under 3 conditions (easy tasks, stress, return to normal). The significance was established at 0.1% for the differences between the groups and between the individuals.

### Results

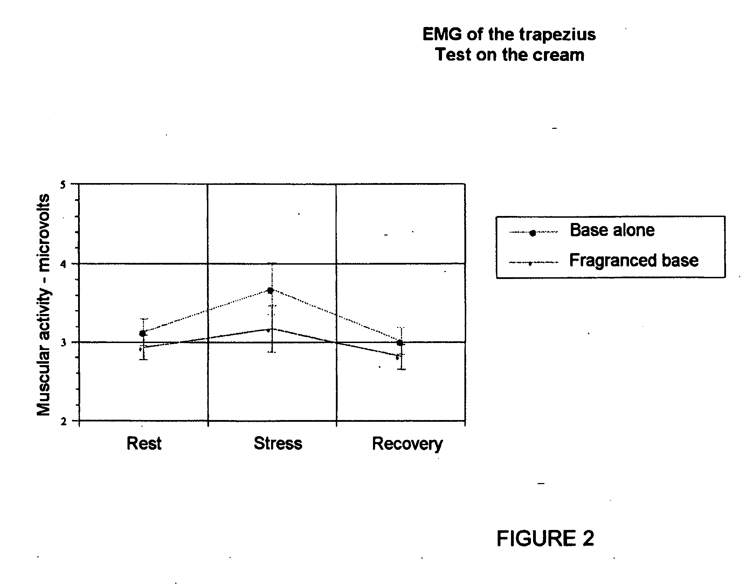

As illustrated in Figure 2, during the stress period, the individuals to whom the fragranced cream was applied showed significantly lower muscular tension values than the individuals to whom the unfragranced cream base was applied. The difference is significant to 0.1%.

The muscle-relaxing effect of the fragrancing composition used according to the invention was thus demonstrated.

### Example 2: Demonstration of the in vivo anti-wrinkle effect

The study was performed on two paired groups of 53 individuals each, applying for 2 weeks: for the first group (A), a cosmetic composition (A) containing the fragrance subjected to the test of Example 1, and, for the second group (B), an identical cosmetic composition (B) but containing a standard fragrance of pleasant odour.

A sealed questionnaire was given to the individuals at the end of the treatment to allow them to self-assess the performance qualities of the product. It comprised several assertions with respect to which the individuals had to indicate whether they were entirely, relatively, relatively not or not at all in agreement.

The results are collated in Table 1 below:

**Table 1**

| Perception of the fragranced compositions | | | |
|---|---|---|---|
| | Total % of "entirely in agreement" and "relatively in agreement" | | Significance |
| | Composition A | Composition B | |
| Makes the skin smooth | 92 | 70 | p < 0.05 |
| Makes the skin firm | 86 | 68 | p < 0.05 |
| Gives the skin tonicity | 91 | 77 | p < 0.05 |
| | % of "entirely in agreement" | | |
| Prevents ageing | 34 | 17 | p < 0.05 |

These results demonstrate a perceived efficacy on the signs of ageing, which is greater for composition A containing a fragrance according to the invention than for composition B containing a standard fragrance.

Moreover, between 70% and 80% of the individuals of groups A and B assessed the fragrances contained in compositions A and B as being pleasant, of good intensity and suitable for anti-wrinkle care (non-significant difference between the groups).

### Example 3: Cosmetic compositions

The compositions below were prepared in a manner that is conventional to those skilled in the art. All the percentages given are percentages by weight. The fragrance has the composition indicated in Example 1.

### A. Anti-wrinkle cream

| FATTY PHASE | |
|---|---|
| Polyethylene glycol stearate | 1% |
| Glyceryl stearate | 2% |
| Cetyl alcohol | 1% |
| Stearic acid | 2% |
| Apricot oil | 5% |
| Myristyl myristate | 5% |
| Cyclopentadimethylsiloxane | 5% |
| Fragrance | 0.5% |
| | |

| AQUEOUS PHASE | |
|---|---|
| Water | qs 100% |
| Preserving agents | qs |
| Triethanolamine | qs pH 6.5 |
| Glycerol | 5 |
| Plant extracts (active agents) | 5 |

### B. Makeup-removing milk

| FATTY PHASE | |
|---|---|
| Isopropyl palmitate | 8% |
| Mineral oil | 17% |
| Fragrance | 0.3% |
| | |

| AQUEOUS PHASE | |
|---|---|
| Carbomer | 1% |
| Water | qs 100% |
| Glycerol | 5% |
| Sodium hydroxide | qs pH 6.5 |

### C. Tonic lotion

| | |
|---|---|
| Water | qs 100% |
| Glycerol | 5% |
| Preserving agents | qs |
| Plant extract | 3% |
| Fragrance | 0.01% |

### D. Foaming composition

| | |
|---|---|
| A) Water | qs 100% |
| Glycerol | 3% |
| Preserving agents | qs |
| | |
| B) Sodium lauryl ether sulphate | 15% |
| Alkylpolyglucoside | 6% |
| | |
| C) Acrylate/steareth/methacrylate copolymer | 5% |
| | |
| D) Water | 1.2% |
| Sodium hydroxide | 0.21% |

### E. Fragrancing cream

| | |
|---|---|
| Liquid paraffin | 43% |
| Polydimethylsiloxane | 0.3% |
| Paraffin | 6% |
| Ozokerite | 7% |
| | |
| Liquid paraffin | 1% |
| | |
| Nylon powder | 20% |
| | |
| Fragrance | 20% |
| Polytrap | 3% |
| Nylon powder | 0.5% |

The above compositions make it possible to relax the lines of the face, to smooth out the surface of the skin and to reduce the depth of the expression wrinkles and fine lines.

## Claims

1. Cosmetic process via inhalation of a fragrancing composition for preventing and/or smoothing out expression wrinkles and/or for relaxing the lines of the face, comprising the topical application to facial skin of a cosmetic composition containing, in a physiologically acceptable medium said fragrancing composition, said fragrancing composition containing from 5% to 10% by weight of essential oils and from 90% to 95% by weight of a mixture comprising:
(a) from 10% to 15% by weight of alcohols,
(b) from 10% to 15% by weight of aldehydes,
(c) from 25% to 30% by weight of esters,
(d) from 20% to 30% by weight of at least one compound chosen from: musks and ketones, and
(e) from 5% to 10% by weight of solvents,
said essential oils being chosen among the essential oils of lemon, of orange, of aniseed, of bergamot, of rose, of geranium, of ginger, of neroli, of basil, of rosemary, of cardamom, of camphor, of cedar, of camomile, of sandalwood, and of sage, and mixtures thereof and a mixture of bergamot, mandarin and ylang-ylang,
said compounds (a) to (d) being chosen among geraniol, geranyl acetate, farnesol, borneol, bornyl acetate, linalool, linalyl acetate, linalyl propionate, linalyl butyrate, tetrahydrolinalool, citronellol, citronellyl acetate, citronellyl formate, citronellyl propionate, dihydromyrcenol, dihydromyrcenyl acetate, tetrahydromyrcenol, terpineol, terpinyl acetate, nopol, nopyl acetate, nerol, neryl acetate, 2-phenylethanol, 2-phenylethyl acetate, benzyl alcohol, benzyl acetate, benzyl salicylate, styrallyl acetate, benzyl benzoate, amyl salicylate, dimethylbenzylcarbinol, trichloromethylphenylcarbinyl acetate, p-tert-butylcyclohexyl acetate, isononyl acetate, vetiveryl acetate, vetiverol, α-hexylcinnamaldehyde, 2-methyl-3-(p-tert-butylphenyl)-propanal, 2-methyl-3-(p-isopropylphenyl)propanal, 3-(p-tert-butylphenyl)propanal, 2,4-dimethylcyclohex-3-enylcarboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyltetrahydropyran, 3-carboxymethyl-2-pentylcyclopentane, 2-n-heptylcyclopentanone, 3-methyl-2-pentyl-2-cyclopentenone, menthone, carvone, tagetone, geranyl acetone, n-decanal, n-dodecanal, 9-decen-l-ol, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepinonitrile, aubepine, heliotropin, coumarin, eugenol, vanillin, diphenyl ether, citral, citronellal, hydroxycitronellal, damascone, ionones, methylionones, isomethylionones, solanone, irones, cis-3-hexenol and its esters, indane musks, tetralin musks, isochroman musks, macrocyclic ketones, macrolactone musks and ethylene brassylate, and mixtures thereof
and said solvents being chose among ethanol, isopropanol, diethylene glycol monoethyl ether, dipropylene glycol, diethyl phthalate, triethyl citrate and isopropyl myristate,
this fragrancing composition resulting in, when it is introduced in a proportion of 0.5% by weight into 2 ml of a topical preparation applied to the part of the skin located between the nose and the top lip of a human individual subjected to stress, a reduction in the muscular activity of the trapezius muscle, measured by electromyography, compared with the unfragranced topical preparation tested under the same conditions.

2. Process according to Claim 1, **characterized in that** the cosmetic composition is a skincare, cleansing or makeup composition.

3. Process according to Claim 1 or 2, **characterized in that** the said fragrancing composition represents from 0.01% to 25% of the total weight of the cosmetic composition.

4. Process according to any one of Claims 1 to 3, **characterized in that** the said cosmetic composition also contains at least one compound chosen from: desquamating agents; moisturizers; depigmenting or propigmenting agents; anti-glycation agents; NO-synthase inhibitors; agents for stimulating the synthesis of dermal or epidermal macromolecules and/or for preventing their degradation; agents for stimulating fibroblast and/or keratinocyte proliferation or for stimulating keratinocyte differentiation; muscle relaxants; tensioning agents; antipollution agents and/or freeradical scavengers; agents acting on the capillary circulation; agents acting on the energy metabolism of cells; and mixtures thereof.

5. Process according to Claim 4, **characterized in that** the said compound is chosen from: manganese gluconate; an extract of wild yam containing diosgenin; a ceramide; a retinoid chosen from: retinyl palmitate, retinyl caprylate and retinol; and acetyl trifluoromethylphenyl valylglycine.

6. Process according to any one of Claims 1 to 5, **characterized in that** the said composition is applied to individuals with expression wrinkles and fine lines.

7. Process according to any one of Claims 1 to 6, **characterized in that** the said composition is applied to the part of the skin located between the nose and the mouth.

## Patentansprüche

1. Kosmetisches Verfahren über Inhalation einer wohlriechenden Zusammensetzung zur Verhinderung und/oder Glättung von Ausdrucksfalten und/oder zur Entspannung der Gesichtskonturen, umfassend die topische Aufbringung einer kosmetischen Zusammensetzung, enthaltend die wohlriechende Zusammensetzung in einem physiologisch verträglichen Medium, auf die Gesichtshaut, wobei die wohlriechende Zusammensetzung 5 Gew.-% bis 10 Gew.-% ätherische Öle und 90 Gew.-% bis 95 Gew.% eines Gemisches, umfassend:
(a) 10 Gew.-% bis 15 Gew.-% Alkohole,
(b) 10 Gew.-% bis 15 Gew.-% Aldehyde,
(c) 25 Gew.-% bis 30 Gew.-% Ester,
(d) 20 Gew.-% bis 30 Gew.-% mindestens einer Verbindung, welche aus Moschus und Ketonen ausgewählt ist, und
(e) 5 Gew,% bis 10 Gew.-% Lösungsmittel,
enthält, wobei die ätherischen Öle unter den ätherischen Ölen von Zitrone, Orange, Anis, Bergamotte, Rose, Geranie, Ingwer, Pomeranze, Basilikum, Rosmarin, Kardamom, Kampfer, Zeder, Kamille, Sandelholz und Salbei und Gemischen davon und einem Gemisch von Bergamotte, Mandarine und Ylang-Ylang ausgewählt sind,
wobei die Verbindungen (a) bis (d) unter Geraniol, Geranylacetat, Farnesol, Borneol, Bornylacetat, Linalool, Linalylacetat, Linalylpropionat, Linalylbutyrat, Tetrahydrolinalool, Citronellol, Citronellylacetat, Citronellylformiat, Citronellylpropionat, Dihydromyrcenol, Dihydromyrcenylacetat, Tetrahydromyrcenol, Terpineol, Terpinylacetat, Nopol, Nopylacetat, Nerol, Nerylacetat, 2-Phenylethanol, 2-Phenylethylacetat, Benzylalkohol, Benzylacetat, Benzylsalicylat, Styrallylacetat, Benzylbenzoat, Amylsalicylat, Dimethylbenzylcarbinol, Trichlormethylphenylcarbinylacetat, p-tert-Butylcyclohexylacetat, Isononylacetat, Vetiverylacetat, Vetiverol, α-Hexylcinnamaldehyd, 2-Methyl-3-(p-tert-butylphenyl)propanal, 2-Methyl-3-(p-isopropylphenyl)propanal, 3-(p-tert-Butylphenyl)propanal, 2,4-Dimethylcyclohex-3-enylcarboxaldehyd, Tricyclodecenylacetat, Tricyclodecenylpropionat, 4-(4-Hydroxy-4-methylpentyl)-3-cyclohexencarboxaldehyd, 4-(4-Methyl-3-pentenyl)-3-cyclohexencarboxaldehyd, 4-Acetoxy-3-pentyltetrahydropyran, 3-Carboxymethyl-2-pentylcyclopentan, 2-n-Heptylcyclopentanon, 3-Methyl-2-pentyl-2-cyclopentenon, Menthon, Carvon, Tageton, Geranylaceton, n-Decanal, n-Dodecanal, 9-Decen-1-ol, Phenoxyethylisobutyrat, Phenylacetaldehyddimethylacetal, Phenylacetaldehyddiethylacetal, Geranonitril, Citronellonitril, Cedrylacetat, 3-Isocamphylcyclohexanol, Cedrylmethylether, Isolongifolanon, Aubepinonitril, Aubepin, Hellotropin, Coumarin, Eugenol, Vanillin, Diphenylether, Citral, Citronellal, Hydroxycitronellal, Damascon, Iononen, Methyliononen, Isomethyliononen, Solanon, Ironen, cis-3-Hexenol und seinen Estern, Indanmoschus, Tetralinmoschus, Isochromanmoschus, makrocyclischen Ketonen, Makrolactonmoschus und Ethylenbrassylat und Gemischen davon ausgewählt sind,
und wobei die Lösungsmittel unter Ethanol, Isopropanol, Diethylenglykolmonoethylether, Dipropylenglykol, Diethylphthalat, Triethylcitrat und Isopropylmyristat ausgewählt sind,
wobei diese wohlriechende Zusammensetzung, wenn sie in einem Anteil von 0,5 Gew.-% in 2 ml einer topischen Zubereitung eingebracht wird, welche auf den Teil der Haut aufgebracht wird, welcher zwischen der Nase und der Oberlippe eines Menschen lokalisiert ist, der Stress ausgesetzt ist, in einer Verringerung der Muskelaktivität des Trapezius-Muskels resultiert, gemessen durch Elektromyographie, im Vergleich mit der topischen Zubereitung, welche nicht wohlriechend gemacht wurde, wobei unter den gleichen Bedingungen getestet wurde.

2. Verfahren gemäß Anspruch 1, **dadurch gekennzeichnet dass** die kosmetische Zusammensetzung eine Hautpflege-, Reinigungs- oder Makeup-Zusammensetzung ist.

3. Verfahren gemäß Anspruch 1 oder 2, **dadurch gekennzeichnet dass** die wohlriechende Zusammensetzung 0,01 % bis 25 % des Gesamtgewichts der kosmetischen Zusammensetzung darstellt.

4. Verfahren gemäß einem der Ansprüche 1 bis 3, **dadurch gekennzeichnet dass** die kosmetische Zusammensetzung auch mindestens eine Verbindung enthält, welche ausgewählt ist aus Desquamatierungsmitteln; feuchtigkeitsspendenden Mitteln; Depigmentierungs- oder Propigmentierungsmitteln; Antiglycationsmitteln; NO-Synthase-Inhibitoren; Mitteln zur Stimulierung der Synthese von dermalen oder epidermalen Makromolekülen und/oder zur Verhinderung ihres Abbaus; Mitteln zur Stimulierung von Fibroblasten- und/oder Keratinozytenprolifezation oder zur Stimulierung von Keratinozytendifferenzierung; Muskelrelaxantia; Straffungsmittel:
Mitteln gegen Verschmutzung und/oder Radikalfängern, Mitteln, welche auf den Kapillarkreislauf einwirken; Mitteln, welche auf den Energiemetabolismus von Zellen einwirken; und Gemischen davon.

5. Verfahren gemäß Anspruch 4, **dadurch gekennzeichnet dass** die Verbindung ausgewählt ist aus Mangangluconat; einem Extrakt von wilder Yamswurzel, der Diosgenin enthält; einem Ceramid; einem Retinoid, welches aus Retinylpalmitat, Retinylcaprylat und Retinol ausgewählt ist; und Acetyltrifluormethylphenylvalylglycin.

6. Verfahren gemäß einem der Ansprüche 1 bis 5, **dadurch gekennzeichnet dass** die Zusammensetzung auf Individuen mit Ausdrucksfalten und feinen Konturen aufgebracht wird,

7. Verfahren gemäß einem der Ansprüche 1 bis 6, **dadurch gekennzeichnet dass** die Zusammensetzung auf den Teil der Haut, der zwischen der Nase und dem Mund lokalisiert ist, aufgebracht wird.

## Revendications

1. Procédé cosmétique par inhalation d'une composition parfumante pour prévenir et/ou lisser des rides d'expression et/ou pour détendre les traits du visage, comprenant l'application topique, sur la peau du visage, d'une composition cosmétique contenant, dans un milieu physiologiquement acceptable, ladite composition parfumante, ladite composition parfumante contenant de 5 % à 10 % en poids d'huiles essentielles et de 90 % à 95 % en poids d'un mélange comprenant :
(a) de 10 % à 15 % en poids d'alcools,
(b) de 10 % à 15 % en poids d'aldéhydes,
(c) de 25 % à 30 % en poids d'esters,
(d) de 20 % à 30 % en poids d'au moins un composé choisi parmi : les muscs et les cétones, et
(e) de 5 % à 10 % en poids de solvants,
lesdites huiles essentielles étant choisies parmi les huiles essentielles de citron, d'orange, d'anis, de bergamote, de rose, de géranium, de gingembre, de néroli, de basilic, de romarin, de cardamome, de camphre, de cèdre, de camomille, de bois de santal et de sauge, et des mélanges de celles-ci et un mélange de bergamote, de mandarine et d'ylang-ylang,
lesdits composés (a) à (d) étant choisis parmi le géraniol, l'acétate de géranyle, le farnésol, le bornéol, l'acétate de bornyle, le linalol, l'acétate de linalyle, le propionate de linalyle, le butyrate de linalyle, le tétrahydrolinanol, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzylcarbinol, l'acétate de trichlorométhylphénylcarbinyle, l'acétate de p-tert-butylcyclohexyle, l'acétate d'isononyle, l'acétate de vétivéryle, le vétivérol, le α-hexylcinnamaldéhyde, le 2-méthyl-3-(p-tert-butylphényl)-propanal, le 2-méthyl-3-(p-isopropylphényl)propanal, le 3-(p-tert-butylphényl)propanal, le 2,4-diméthylcyclohex-3-énylcarboxaldéhyde, l'acétate de tricyclodécényle, le propionate de tricyclodécényle, le 4-(4-hydroxy-4-méthylpentyl)-3-cyclohexènecarboxaldéhyde, le 4-(4-méthyl-3-pentényl)-3-cyclohexènecarboxaldéhyde, le 4-acétoxy-3-pentyltétrahydropyranne, le 3-carboxyméthyl-2-pentylcyclopentane, la 2-n-heptylcyclopentanone, la 3-méthyl-2-pentyl-2-cyclopenténone, la menthone, la carvone, la tagétone, la géranyl acétone, le n-décanal, le n-dodécanal, le 9-décén-1-ol, l'isobutyrate de phénoxyéthyle, le phénylacétaldéhyde diméthyl acétal, le phénylacétaldéhyde diéthyl acétal, le géranonitrile, le citronellonitrile, l'acétate de cédryle, le 3-isocamphylcyclohexanol, l'éther méthylique de cédryle, l'isolongifolanone, l'aubépinonitrile, l'aubépine, l'héliotropine, la coumarine, l'eugénol, la vanilline, l'éther diphénylique, le citral, le citronellal, l'hydroxycitronellal, la damascone, les ionones, les méthylionones, les isométhylionones, la solanone, les irones, le cis-3-hexénol et ses esters, les muscs indane, les muscs tétraline, les muscs isochromane, les cétones macrocycliques, les muscs macrolactone et le brassylate d'éthylène, et des mélanges de ceux-ci
et lesdits solvants étant choisis parmi l'éthanol, l'isopropanol, l'éther monoéthylique de diéthylèneglycol, le dipropylèneglycol, le phtalate de diéthyle, le citrate de triéthyle et le myristate d'isopropyle,
cette composition parfumante conduisant, lorsqu'elle est introduite en une proportion de 0,5 % en poids dans 2 ml d'une préparation topique appliquée sur la partie de la peau se trouvant entre le nez et la lèvre supérieure d'un individu humain soumis à un stress, à une réduction de l'activité musculaire du muscle trapèze, mesurée par électromyographie, par rapport à la préparation topique non parfumée testée dans les mêmes conditions.

2. Procédé selon la revendication 1, **caractérisé en ce que** la composition cosmétique est une composition pour le soin de la peau, le démaquillage ou le maquillage.

3. Procédé selon la revendication 1 ou 2, **caractérisé en ce que** ladite composition parfumante représente de 0,01 % à 25 % du poids total de la composition cosmétique.

4. Procédé selon l'une quelconque des revendications 1 à 3, **caractérisé en ce que** ladite composition cosmétique contient aussi au moins un composé choisi parmi : les agents desquamants ; les hydratants ; les agents dépigmentants ou propigmentants ; les agents anti-glycation ; les inhibiteurs de la NO-synthase ; les agents pour stimuler la synthèse de macromolécules dermiques ou épidermiques et/ou pour prévenir leur dégradation ; les agents pour stimuler la prolifération des fibroblastes et/ou des kératinocytes ou pour stimuler la différentiation des kératinocytes ; les myorelaxants ; les agents tenseurs ; les agents antipollution et/ou les épurateurs de radicaux libres; les agents agissant sur la circulation capillaire; les agents agissant sur le métabolisme énergétique des cellules ; et des mélanges de ceux-ci.

5. Procédé selon la revendication 4, **caractérisé en ce que** ledit composé est choisi parmi : le gluconate de manganèse ; un extrait d'igname sauvage contenant de la diosgénine ; un céramide ; un rétinoïde choisi parmi : le palmitate de rétinyle, le caprylate de rétinyle et le rétinol ; et l'acétyl trifluorométhylphényl valylglycine.

6. Procédé selon l'une quelconque des revendications 1 à 5, **caractérisé en ce que** ladite composition est appliquée à des personnes ayant des rides d'expression et des ridules.

7. Procédé selon l'une quelconque des revendications 1 à 6, **caractérisé en ce que** ladite composition est appliquée sur la partie de la peau se trouvant entre le nez et la bouche.
